# EUROPÄISCHE PATENTSCHRIFT

(11) **EP 1 474 437 B1**
(45) Veröffentlichungstag und Bekanntmachung des Hinweises auf die Patenterteilung: **23.12.2009**
(21) Anmeldenummer: 03704494.8
(22) Anmeldetag: 30.01.2003
(51) Int. Cl.: C07J 63/00, A61K 31/56

(54) **VERBESSERTES VERFAHREN ZUR GEWINNUNG VON BETULINSÄURE**
IMPROVED METHOD FOR OBTAINING BETULINIC ACID
PROCEDE DE PRODUCTION AMELIORE D'ACIDE BETULINIQUE

(30) Priorität: 02.02.2002 DE 10204271
(43) Veröffentlichungstag der Anmeldung: 10.11.2004
(73) Patentinhaber: Boehringer Ingelheim Pharma GmbH & Co.KG, 55216 Ingelheim am Rhein (DE)
(72) Erfinder: SAUTER, Markus, 55457 GENSINGEN (DE)
(86) Internationale Anmeldenummer: PCT/EP2003/000905
(87) Internationale Veröffentlichungsnummer: WO 2003/066659

(56) Entgegenhaltungen:
- WO-A-01/10885
- DE-A- 19 713 768
- US-B1- 6 264 998
- ROBERTSON A ET AL: "POLYTERPENOID COMPOUNDS PART I. BETULIC ACID FROM CORNUS FLORIDA, L" JOURNAL OF THE CHEMICAL SOCIETY, CHEMICAL SOCIETY, LONDON, GB, 1939, Seiten 1267-1273, XP001025917 ISSN: 0368-1769
- BRUCKNER V ET AL: "OCCURRENCE OF BETULINIC ACID IN THE BARK OF THE PLANE TREE" JOURNAL OF THE CHEMICAL SOCIETY, CHEMICAL SOCIETY, LONDON, GB, 1948, Seiten 948-951, XP001080318 ISSN: 0368-1769 in der Anmeldung erwähnt

## Beschreibung

Die Erfindung betrifft ein verbessertes Verfahren zur Gewinnung von hochreiner, kristalliner Betulinsäure aus einem methanolischen Extrakt von gemahlener Platanenrinde.

### Hintergrund der Erfindung

### Betulinsäure ist 3β-Hydroxy-lup-20(29)-en-28-säure der Formel

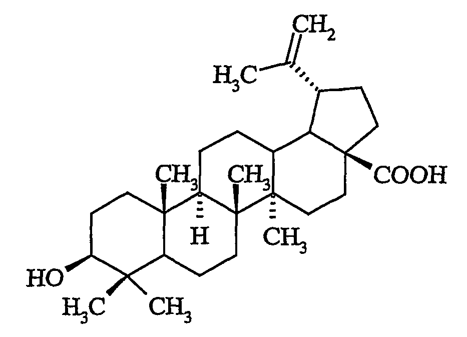

Es ist bekannt, dass Betulinsäure eine Wirkung gegen das Wachstum von Melanomzellen (z.B. Pisha et al., Nature Medicine 1, 1995, 1046 ff) sowie gegen andere Krebszellen (z.B. Sunder et al., US Patent US 6,048,847) aufweist. Desweiteren sollen seine Amide gegen HIV eingesetzt werden können (z.B. Evers et al., J. Med. Chem. 39, 1996, 1056 ff; oder Soler et al., J. Med. Chem. 39, 1996, 1069 ff). Demzufolge besteht ein großer Bedarf an Betulinsäure.

Neben der synthetischen Herstellung (z.B. L. Ruzicka et al., Helv. Chim. Acta 21, 1938, 1076 ff) kann Betulinsäure aus verschiedenen Pflanzen, insbesondere Bäumen gewonnen werden, so zum Beispiel aus der Rinde von *Picramnia pentandra* (z.B. Herz et al., Phytochemistry 11, 1972, 3061 ff), aus der Rinde von *Arbutus menziesii* (Robinson et al., Phytochemistry 9, 1970, 907 ff) und aus der Rinde von *Ziziphus mauritiana* (z.B. Pisha et al., Nature Medicine 1, 1995, 1046 ff).

Aus diesen Ausgangsmaterialien lässt sich die Betulinsäure nur schwer isolieren. Aussichtsreicher dagegen erscheint ihre Gewinnung aus der Rinde und/oder Borke der Platane (*Platanus acerifolia*). In der DE 197 13 768 wird ein Verfahren zur Gewinnung von Betulinsäure vorgeschlagen, wobei ein aus Platanenborken gewonnenes Pulver mit einem mittelpolaren Lösungsmittel, wie zum Beispiel Dichlormethan, Chloroform oder Diethylether extrahiert werden.

Dieses Verfahren ist jedoch für eine industrielle Gewinnung von großen Mengen an Betulinsäure nicht geeignet, da sehr große Volumina der mittelpolaren Lösungsmittel eingesetzt werden müssen, um die Betulinsäure zu extrahieren (Einsatz von 7 Litern Dichlormethan auf 150 g Platenenborken-Pulver).

Bruckner et al., J. Chem. Soc. 1948, 948-951 beschreiben ein Verfahren zur Gewinnung von Betulinsäure aus Platanenborken, wobei die gemahlenen Borken mit Methanol extrahiert werden, der gewonnene Extrakt eingeengt wird, und das Konzentrat in Gegenwart von Kohle mehrfach aus Methanol umkristallisiert wird. Die so gewonnene Betulinsäure weist jedoch noch eine Vielzahl von Verunreinigungen auf.

Der vorliegenden Erfindung lag daher die Aufgabe zugrunde ein verbessertes Verfahren zur großtechnischen Gewinnung von hochreiner, kristalliner Betulinsäure aus Platanenborken und/oder Platanenrinden bereitzustellen, welches die Nachteile der bekannten Verfahren vermeidet.

### Detaillierte Beschreibung der Erfindung

Überraschenderweise wurde nun gefunden, dass hochreine, kristalline Betulinsäure aus einem methanolischen Extrakt von gemahlener Platanenborke und/oder Platanenrinde durch Einengen des Extraktes und Kristallisation aus einem Alkohol erhalten werden kann, wenn man den Rückstand der methanolischen Extraktion vor der Kristallisation mit einem unpolaren Verdünnungsmittel wäscht.

Gegenstand der Erfindung ist somit ein Verfahren zur Gewinnung von hochreiner, kristalliner Betulinsäure aus einem methanolischen Extrakt von gemahlener Platanenborke und/oder Platanenrinde durch Einengen des Extraktes und Kristallisation aus einem Alkohol, **dadurch gekennzeichnet, dass** man den Rückstand vor der Kristallisation mit einem unpolaren Verdünnungsmittel wäscht.

Der Begriff "Betulinsäure" wie er vor- und nachstehend verwendet wird, umfasst sowohl Betulinsäure als solche sowie ihre Hydrate und Solvate, vorzugsweise Betulinsäure, welche mit 1 bis 2 Equivalenten eines Alkohols, insbesondere einem Equivalent Ethanol solvatisiert ist.

Ein weiterer Gegenstand der Erfindung ist die hochreine kristalline Betulinsäure, welche mit einem Equivalent Ethanol solvatisiert ist.

Als besonders geeignete Platanen haben sich die Abendländische Platane (*Platanus occidentalis*) erwiesen.

Als Alkohole zur Umkristallisation wird Ethanol verwendet.

Als unpolare Verdünnungsmittel eignen sich in der Regel aliphatische, cycloaliphatische oder aromatische Kohlenwasserstoffe oder Gemische derselben, vorzugsweise aliphatische Kohlenwasserstoffe mit 5 bis 8 Kohlenstoffatomen, insbesondere Pentan, Hexan oder Heptan, ganz besonders bevorzugt n-Hexan, cycloaliphatische Kohlenwasserstoffe mit 5 bis 8 Kohlenstoffatomen, insbesondere Cyclopentan, Cyclohexan, Methylcyclohexan, oder aromatische Kohlenwasserstoffe mit 6 bis 9 Kohlenstoffatomen, insbesondere Toluol oder Xylol.

Sofern vor- oder nachstehend der Einfachheit halber das Verhältnis zweier Stoffe in der Form "x bis y-fache Menge" angegeben ist, wobei x und y jeweils für die Unter- und Obergrenze der angegebenen Menge stehen, bezieht sich diese Angabe auf "x bis y" Gewichtsteile des einen Stoffes bezogen auf ein (1) Gewichtsteil des anderen Stoffes.
(A) Erfindungsgemäß werden nacheinander folgende Schritte zum Waschen und Isolieren des Rückstandes des methanolischen Extraktes durchgeführt.
   (i) Erhitzen des Rückstandes in einem unpolaren Verdünnungsmittel;
   (ii) Abtrennen des Rückstandes von dem Verdünnungsmittel;
   (iii) Erhitzen des Rückstandes in Ethanol, Klärung der ethanolischen Phase durch Zusatz von Aktivkohle und Abtrennen der Aktivkohle von der ethanolischen Phase bei einer Temperatur zwischen 60 und 95 °C; und
   (iv) Kristallisation und Abtrennen der Betulinsäure aus ethanolischen Phase.
(B) Erfindungsgemäß werden die gemahlene Platanenrinde einmal mit einer 2 bis 5fachen Menge Methanol für 0,5 bis 5,0 Stunden zum Sieden erhitzt extrahiert, den erhaltenen Extrakt bei einer Temperatur zwischen 40 und 60 °C filtriert, auf 30 bis 70 % seines Volumens einengt und bei einer Temperatur zwischen - 10 und + 10 °C kristallisisiert. Bevorzugte Ausführungsformen der Erfindung sind:
(C) Verfahren, wobei man den Rückstand des methanolischen Extraktes in einer 2 bis 8fachen Menge eines aliphatischen Kohlenwasserstoffes, vorzugsweise Cyclohexan, Methylcyclohexan oder *n*-Hexan, insbesondere *n*-Hexan aufnimmt und das Gemisch zum Sieden erhitzt.
(D) Verfahren, wobei man in Schritt (ii) den Rückstand durch Abschleudern oder Abnutschen von dem Verdünnungsmittel abtrennt.
(E) Verfahren, wobei man in Schritt (iii) den Rückstand in einer 20 bis 100fachen, vorzugsweise 30 bis 80fachen, insbesondere 40 bis 60fachen Menge an Ethanol aufnimmt und zum Sieden erhitzt.
(F) Verfahren, wobei man in Schritt (iv) die ethanolische Phase auf Temperaturen von -10 bis + 10 °C, vorzugsweise von 0 bis + 5 °C abkühlt, die ausfallende Betulinsäure durch Abschleudern oder Abnutschen abtrennt und trocknet.
(G) Verfahren, wobei die erhaltene Betulinsäure als Monoethanolat vorliegt.

Besonders bevorzugt ist die hochreine, kristalline Betulinsäure, welche mit einem Equivalent Ethanol solvatisiert ist, insbesondere das Betulinsäure Ethanolat der Formel

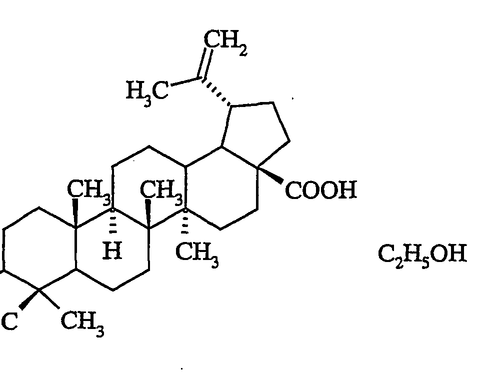

In einer ganz besonders bevorzugten Ausführungsform des erfindungsgemäßen Verfahrens wird das Platanenrinde-Pulver in der 2 bis 5fachen, vorzugsweise 3 bis 4fachen Menge an Methanol suspendiert und 1 bis 5 Stunden, insbesondere etwa 2 Stunden, vorzugsweise unter Rückfluss erhitzt. Die Suspension wird bei 40 bis 60 °C, insbesondere bei etwa 50 °C filtriert, der Methanol-Extrakt auf 60 bis 40 %, insbesondere etwa 50 % seines Volumens eingedampft. Der konzentrierte Extrakt wird auf 0 bis 10 °C, insbesondere etwa 5 °C abgekühlt, abfiltriert und mit kaltem Methanol gewaschen. Der Rückstand wird vorzugsweise im Vakuum bei 40 bis 70 °C, insbesondere bei etwa 50°C getrocknet.

Man erhält so die rohe Betulinsäure als farbloses Pulver (etwa 2,0 bis 2,5 % berechnet auf Platanenrinde). Diese wird in der 4 bis 8fachen, insbesondere 5 bis 6fachen Menge an *n-*Hexan, Cyclohexan oder Methylcyclohexan suspendiert und 0,5 bis 2 Stunden, insbesondere 1 Stunde, insbesondere unter Rückfluss erhitzt. Die erhaltene Suspension wird abgeschleudert oder abgesaugt, mit der 1,5 bis 4,5fachen, insbesondere etwa 3fachen Menge an heißem *n-*Hexan, Cyclohexan oder Methylcyclohexan nachgewaschen, und der Rückstand über Nacht bei etwa 40°C, vorzugsweise *in vacuo* getrocknet.

Die so erhaltene Betulinsäure wird in der 30 bis 50fachen Menge an Ethanol in der Hitze gelöst. Es wird Aktivkohle zugegeben und heiß abfiltriert. Beim Abkühlen setzt Kristallisation ein. Das Gemisch wird auf 0 bis 10 °C, insbesondere 5°C abgekühlt, abgesaugt oder abgeschleudert und mit kaltem Ethanol nachgewaschen.
Man erhält hochreine, kristalline Betulinsäure, welche mit einem mol Ethanol solvatisiert ist.

Das nachfolgende Beispiel dient der Illustration eines exemplarisch durchgeführten Verfahrens zur Gewinnung der Betulinsäure. Es ist lediglich als eine mögliche, exemplarisch dargestellte Vorgehensweisen zu verstehen, ohne die Erfindung auf seinen Inhalt zu beschränken.

### Beispiel

### 1A Extraktion der Platanenrinde

4500 g Platanenrinden-Pulver (über Schneid-Mühle gemahlen) werden in 151 Methanol suspendiert und 2 h am Rückfluss gekocht. Die Suspension wird bei etwa 50 °C filtriert, der Methanol-Extrakt auf 50% seines Volumens eingedampft (7,51). Der konzentrierte Extrakt wird auf 5°C abgekühlt, abfiltriert und mit 21 kaltem Methanol gewaschen. Der Rückstand wird im Vakuum bei 50°C getrocknet.
Man erhält 104 g rohe Betulinsäure als farbloses Pulver (2,3 % berechnet auf Platanenrinde). HPLC: 90,6 %

### 1B Ausrühren aus unpolarem Lösungsmittel

93 g Betulinsäure erhalten nach Beispiel **1A** werden in 600 ml *n*-Hexan suspendiert und 1 Stunde am Rückfluss gekocht. Die Suspension wird über eine Filternutsche abgesaugt, mit 300 ml heißem *n*-Hexan nachgewaschen, und der Rückstand über Nacht bei 40°C *in vacuo* getrocknet.
Man erhält 89,5 g (96,2 %) Betulinsäure als farbloses Pulver. HPLC: 94,75 %
Analog können auch Cyclohexan, Methylcyclohexan oder Gemische dieser Lösungsmittel eingesetzt werden.

### 1C Umkristallisation aus Ethanol

89 g Betulinsäure erhalten nach Beispiel **1B** werden in 41 Ethanol am Rückfluss erhitzt und gelöst. Es werden 40 g Aktivkohle zugegeben und über eine Filternutsche heiß abfiltriert. Nach Abkühlen auf Raumtemperatur setzt Kristallisation ein. Das Gemisch wird auf 5°C abgekühlt, abgesaugt und mit 500 ml kaltem Ethanol nachgewaschen. Man erhält 63,5 g (71,3 %) kristalline Betulinsäure, welche mit 1 mol Ethanol solvatisiert ist. HPLC: 93,8 % (Flächenprozent 100%); Schmelzpunkt= 296-298°C, [a]D = +8, c= 0,9 in Pyridin.

### 1D Aufarbeitung der Mutterlauge

41 der ethanolischen Mutter- und Waschlauge aus Beispiel **1C** werden *in vacuo* auf 11 eingeengt, bei 5°C abgesaugt und mit kaltem Ethanol gewaschen.
Man erhält 20 g (22,5 %) Betulinsäure. HPLC: 96,5 %

## Patentansprüche

1. Verbessertes Verfahren zur Gewinnung von hochreiner, kristalliner Betulinsäure aus einem methanolischen Extrakt von gemahlener Platanenrinde und/oder Platanenborke durch Einengen des Extraktes und Kristallisation aus einem Alkohol, **dadurch gekennzeichnet, dass**
a) man die gemahlene Platanenrinde einmal mit einer 2 bis 5-fachen Menge Methanol für 0,5 bis 5,0 Stunden zum Sieden erhitzt extrahiert, den erhaltenen Extrakt bei einer Temperatur zwischen 40 und 60 °C filtriert, auf 30 bis 70 % seines Volumens einengt und bei einer Temperatur zwischen - 10 und + 10 °C kristallisisiert und
b) nacheinander die folgende Schritte zum Waschen und Isolieren des Rückstandes des methanolischen Extraktes durchführt:
(i) Erhitzen des Rückstandes in einem unpolaren Verdünnungsmittel;
(ii) Abtrennen des Rückstandes von dem Verdünnungsmittel;
(iii) Erhitzen des Rückstandes in Ethanol, Klärung der ethanolischen Phase durch Zusatz von Aktivkohle und Abtrennen der Aktivkohle von der ethanolischen Phase bei einer Temperatur zwischen 60 und 95 °C; und
(iv) Kristallisation und Abtrennen der Betulinsäure aus der ethanolischen Phase.

2. Verfahren nach Anspruch 1, **dadurch gekennzeichnet, dass** man den Rückstand des methanolischen Extraktes in einer 2 bis 8fachen Menge eines aliphatischen Kohlenwasserstoffes, vorzugsweise *n*-Hexan aufnimmt und das Gemisch zum Sieden erhitzt.

3. Verfahren nach Anspruch 1 oder 2, **dadurch gekennzeichnet, dass** man in Schritt (ii) den Rückstand durch Abschleudern von dem Verdünnungsmittel abtrennt.

4. Verfahren nach einem der Ansprüche 1 bis 3, **dadurch gekennzeichnet, dass** man in Schritt (iii) den Rückstand in einer 20 bis 100fachen Menge an Ethanol aufnimmt und zum Sieden erhitzt.

5. Verfahren nach einem der Ansprüche 1 bis 4, **dadurch gekennzeichnet, dass** man in Schritt (iv) die ethanolische Phase auf Temperaturen von -10 bis + 10 °C, vorzugsweise von 0 bis + 5 °C abkühlt, die ausfallende Betulinsäure durch Abschleudern abtrennt und trocknet.

6. Verfahren nach einem der Ansprüche 1 bis 5, **dadurch gekennzeichnet, dass** die so erhaltene Betulinsäure als Monoethanolat vorliegt.

7. Hochreine, kristalline Betulinsäure, **dadurch gekennzeichnet dass** sie mit einem Equivalent Ethanol solvatisiert ist.

8. Hochreine, kristalline Betulinsäure nach Anspruch 7 der Formel

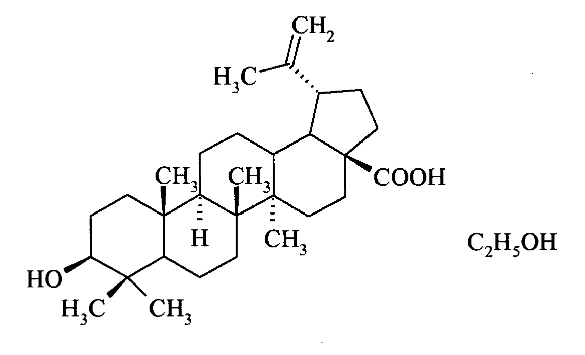

## Claims

1. Improved process for obtaining highly pure, crystalline betulinic acid from a methanolic extract of ground plane cortex and/or plane bark by concentrating the extract and crystallising it from an alcohol, **characterised in that**
a) the ground plane cortex is heated to boiling once with 2 to 5 times the amount of methanol for 0.5 to 5.0 hours, extracted, the extract obtained is filtered at a temperature between 40 and 60°C, evaporated down to 30 to 70 % of its volume and crystallised at a temperature between - 10 and + 10 °C
and
b) the following steps are carried out successively for washing and isolating the residue of the methanolic extract:
(i) heating the residue in a nonpolar diluent;
(ii) separating the residue from the diluent;
(iii) heating the residue in ethanol, clarifying the ethanolic phase by the addition of activated charcoal and separating the activated charcoal from the ethanolic phase at a temperature between 60 and 95°C; and
(iv) crystallising and separating the betulinic acid from the ethanolic phase.

2. Process according to claim 1, **characterised in that** the residue of the methanolic extract is taken up in 2 to 8 times the amount of aliphatic hydrocarbon, preferably *n*-hexane, and the mixture is heated to boiling.

3. Process according to claim 1 or 2, **characterised in that** in step (ii) the residue is separated from the diluent by centrifuging.

4. Process according to one of claims 1 to 3, **characterised in that** in step (iii) the residue is taken up in 20 to 100 times the amount of ethanol and heated to boiling.

5. Process according to one of claims 1 to 4, **characterised in that** in step (iv) the ethanolic phase is cooled to temperatures of -10 to + 10 °C, preferably 0 to + 5 °C, the betulinic acid precipitated is separated off by centrifuging and dried.

6. Process according to one of claims 1 to 5, **characterised in that** the betulinic acid thus obtained is present as the monoethoxide.

7. Highly pure crystalline betulinic acid, **characterised in that** it is solvated with one equivalent of ethanol.

8. Highly pure crystalline betulinic acid according to claim 7 of formula

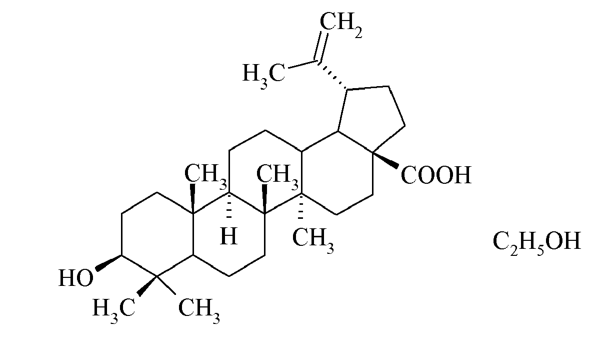

## Revendications

1. Procédé amélioré pour l'obtention d'acide bétulinique cristallin de grande pureté à partir d'un extrait méthanolique d'écorce de platane et/ou de croûte de platane broyée par concentration de l'extrait et cristallisation dans un alcool, **caractérisé en ce que**
a) on extrait l'écorce de platane broyée une fois avec une quantité de méthanol 2 à 5 fois supérieure pendant 0,5 à 5,0 heures en chauffant à ébullition, on filtre l'extrait obtenu à une température entre 40 et 60°C, on le concentre à 30 à 70 % de son volume et on le cristallise à une température entre -10 et +10°C
et
b) on réalise successivement les étapes suivantes pour le lavage et l'isolement du résidu de l'extrait méthanolique :
(i) chauffage du résidu dans un diluant non polaire ;
(ii) séparation du résidu d'avec le diluant ;
(iii) chauffage du résidu dans l'éthanol, clarification de la phase éthanolique par addition de charbon actif et séparation du charbon actif d'avec la phase éthanolique à une température entre 60 et 95°C ; et
(iv) cristallisation et séparation de l'acide bétulinique à partir de la phase éthanolique.

2. Procédé selon la revendication 1 **caractérisé en ce que** l'on reprend le résidu de l'extrait méthanolique dans une quantité 2 à 8 fois supérieure d'un hydrocarbure aliphatique, de préférence le *n*-hexane et on chauffe le mélange à ébullition.

3. Procédé selon la revendication 1 ou 2 **caractérisé en ce que**, dans l'étape (ii), on sépare le résidu d'avec le diluant par centrifugation.

4. Procédé selon l'une des revendications 1 à 3 **caractérisé en ce que**, dans l'étape (iii), on reprend le résidu dans une quantité 20 à 100 fois supérieure d'éthanol et on chauffe à ébullition.

5. Procédé selon l'une des revendications 1 à 4 **caractérisé en ce que,** dans l'étape (iv), on refroidit la phase éthanolique à des températures de -10 à +10°C, de préférence de 0 à +5°C, on sépare par centrifugation l'acide bétulinique précipité et on le sèche.

6. Procédé selon l'une des revendications 1 à 5 **caractérisé en ce que** l'acide bétulinique ainsi obtenu est sous forme de monoéthanolate.

7. Acide bétulinique cristallin de grande pureté **caractérisé en ce qu'**il est solvaté avec un équivalent d'éthanol.

8. Acide bétulinique cristallin de grande pureté selon la revendication 7 de formule
